# EUROPEAN PATENT SPECIFICATION

(11) **EP 4 368 122 B1**
(45) Date of publication and mention of the grant of the patent: **09.12.2026**
(21) Application number: 22855196.6
(22) Date of filing: 20.07.2022
(51) Int. Cl.: A61B 17/00, A61F 2/24, A61F 2/95, A61F 2/966

(54) **HANDLE, CONVEYOR, AND MEDICAL DEVICE**
GRIFF, FÖRDERER UND MEDIZINISCHE VORRICHTUNG
POIGNÉE, TRANSPORTEUR ET DISPOSITIF MÉDICAL

(30) Priority: 09.08.2021 CN 202110908792
(43) Date of publication of application: 15.05.2024
(73) Proprietor: Shanghai Microport Cardioflow Medtech Co., Ltd., Shanghai 201203 (CN)
(72) Inventor: LIU, Shihong, Shanghai 201203 (CN); LIN, Xing, Shanghai 201203 (CN); JI, Lijun, Shanghai 201203 (CN); CHEN, Guoming, Shanghai 201203 (CN)
(74) Representative: Marks & Clerk LLP
(86) International application number: PCT/CN2022/106787
(87) International publication number: WO 2023/016213

(56) References cited:
- CN-A- 110 974 502
- CN-A- 111 214 310
- CN-U- 204 814 284
- CN-U- 206 934 222
- CN-U- 215 651 293
- CN-U- 215 937 493
- US-A1- 2013 116 772
- US-A1- 2014 058 505
- US-A1- 2015 306 358
- US-A1- 2015 335 452
- US-A1- 2020 281 720

## Description

### TECHNICAL FIELD

The present invention relates to the technical field of medical instruments and, more specifically, to a handle, a conveyor and a medical device.

### BACKGROUND

The heart valve is the door to the blood circulation of the heart. Once stenosis or insufficiency occurs, the heart will suffer from insufficient power or failure, causing symptoms such as chest tightness, asthma, general edema, weakness, and chest pain, which are hidden dangers that endanger the life and quality of life of the elderly. With the aging of the population and the development of medical technology, valvular heart disease (VHD) has become the third major cardiovascular disease, seriously endangering human health. At present, traditional surgery is still the preferred treatment for this type of disease. However, for patients who are elderly, have multiple organ diseases, have a history of thoracotomy, and have poor physical recovery function, traditional surgery has high risks and high mortality. There was not even a chance for some patients to receive a surgery.

Interventional surgery is a brand-new treatment technology developed in recent years and is a minimally invasive surgical treatment. Interventional therapy is for introduction of special medical devices into the human body through a conveyor under the guidance of medical imaging equipment to diagnose and locally treat lesions in the body. Interventional treatment does not require surgery. Compared with traditional surgery, it has the advantages of less trauma, faster recovery, and better results. Interventional surgery can be used to treat heart valve disease or other diseases. The medical devices are different depending on the specific disease. For example, for heart valve disease, the medical device is an artificial valve, and for vascular disease, the medical device can be a medical stent.

The conveyor usually includes a handle and a catheter assembly. The catheter assembly is used to load medical devices and includes an inner tube and an outer tube. The handle is used to connect with the catheter assembly and control the inner tube and/or outer tube to enable the medical device to be introduced into human body. As the power source during the entire interventional surgery, the handle needs to ensure sufficient safety, effectiveness and economy. There are different requirements for the operation of the handle at different stages of interventional surgery. For example, during the process of controlling the backward movement of the outer tube to release a medical implant, the operator may need to suspend the backward movement when the outer tube is moved back to the predetermined position so as to perform other operations such as adjusting the position of the medical implants. Therefore, the structure of the handle needs to be further optimized to meet different usage requirements.

US2015/335452 discloses a stent delivery device having a locking mechanism. The device includes a handle assembly, an elongate inner member, and an elongate outer tubular member surrounding the elongate inner member. The handle assembly includes a handle attached to one of the inner member and the outer tubular member, and an actuator attached to the other of the inner member and the outer tubular member. Actuation of the actuator relative to the handle between a first position and a second position causes axial movement of the outer tubular member relative to the inner member. The locking mechanism has a locked position in which the actuator is prevented from moving from the first position to the second position and an unlocked position in which the actuator is permitted to move from the first position to the second position. US 2020/281720 discloses a prosthetic delivery system including a plurality of concentric shafts and an actuator mechanism for actuating one or more of the concentric shafts. A stop mechanism is coupled to the actuator mechanism to prevent advancement or retraction of at least some of the shafts beyond a predetermined position unless the stop mechanism is released.

### SUMMARY OF THE INVENTION

An object of the present invention is to provide a handle, a conveyor and a medical device. When the conveyor is used to convey and release a medical implant, the movement of the release tube can be suspended when the release tube moves to a predetermined position to meet usage requirements.

In order to achieve the above object, the present invention provides a handle, for controlling a catheter assembly, the catheter assembly comprising an outer tube and an inner tube partially inserted in the outer tube, the outer tube or the inner tube used as a release tube; wherein the handle comprises:
a housing;
a first driving part, for driving the release tube to move in a predetermined direction; and
a limiting assembly, comprising a first limiting mechanism and a second limiting mechanism, the first limiting mechanism partially arranged in the housing and connected to the housing, and the first limiting mechanism configured to be rotatable around an axis of the release tube; the second limiting mechanism arranged in the housing and configured to move with movement of the release tube;
wherein the handle is configured such that, when the first driving part drives the release tube to move in the predetermined direction to a predetermined position, the first limiting mechanism and the second limiting mechanism engage with each other, so that the release tube is prevented from further moving in the predetermined direction; when the first limiting mechanism rotates around the axis of the release tube to cause the first limiting mechanism and the second limiting mechanism to disengage from each other, the first driving part is allowed to drive the release tube to further move in the predetermined direction;
wherein the handle further comprises a first connecting component, wherein the first connecting component is arranged in the housing and is drivingly connected to the first driving part, and the first connecting component is further configured to connect with a proximal end of the release tube; the second limiting mechanism is connected to the first connecting component;
characterized in that the first limiting mechanism comprises a first positioning seat and a first limiting part; wherein the first positioning seat is connected to the housing and is configured to be rotatable around the axis of the release tube; the first limiting part is connected to the first positioning seat and at least partially protrudes from a predetermined end face of the first positioning seat; the second limiting mechanism comprises a second limiting part;
wherein the handle is configured such that when the first limiting part and the second limiting part at least partially align with each other in a circumferential direction, and when the first limiting part and the second limiting part abut against each other, the first limiting mechanism and the second limiting mechanism engage with each other; when the first positioning seat rotates around the axis of the release tube to cause the first limiting part and the second limiting part to be staggered with each other in the circumferential direction, the second limiting mechanism and the first limiting mechanism are disengaged from each other.

Optionally, the first limiting part is movably connected to the first positioning seat and configured to movable along an axial direction of the release tube to change a length of a portion of the first limiting part protruding from the predetermined end face of the first positioning seat.

Optionally, the first positioning seat defines therein a screw hole extending through the axial direction of the release tube, and the first limiting part is a screw rod inserted into the screw hole.

Optionally, the housing defines thereon a first slide slot extending in the circumferential direction; the first limiting mechanism further comprises a first lever having one end connected to the first positioning seat and a further end extending out of the housing through the first slide slot.

Optionally, the first limiting mechanism further comprises a first elastic sleeve sleeved on the first lever, and the first elastic sleeve has an outer wall in contact with a wall of the first slide slot so that the first elastic sleeve is deformed.

Optionally, the first positioning seat has an annular structure defining an inner hole, and a proximal end of the first connecting component passes through the inner hole to connect to the first driving part.

Optionally, a number of the first limiting parts is multiple, and the multiple first limiting parts are arranged sequentially along a circumference of the first positioning seat, and a length of a portion of each first limiting part protruding from the predetermined end face of the first positioning seat is sequentially reduced along a first direction, so that an axial distance from each first limiting part to the second limiting part increases sequentially along the first direction;
wherein the handle is configured such that when the first positioning seat rotates around the axis of the release tube in a second direction to cause one of the first limiting parts to be staggered with the second limiting part in the circumferential direction, another one of the first limiting parts can at least partially align with the second limiting part in the circumferential direction; the second direction is opposite to the first direction.

Optionally, the first driving part is rotatably connected to a proximal end of the housing;
an inner wall of the first driving part is provided with internal threads, the first connecting component comprises a screw rod, a distal end of the screw rod is connected to the proximal end of the release tube, a distal outer surface of the screw rod is connected to the second limiting mechanism, and a proximal outer surface of the screw rod is provided with external threads matching with the internal threads of the first driving part for spiral transmission; or,
the first connecting component comprises a threaded sleeve and a screw rod, the threaded sleeve is connected to an inner wall of the first driving part and is configured to rotate synchronously with the first driving part, an inner wall of the threaded sleeve is provided with internal threads; a distal end of the screw rod is connected to the proximal end of the release tube, a distal outer surface of the screw rod is connected to the second limiting mechanism, and a proximal outer surface of the screw rod is provided with external threads matching with the internal threads of the threaded sleeve for spiral transmission.

Optionally, the housing is provided with an observation window for observing movement of the screw rod.

Optionally, the release tube is the outer tube, and the predetermined direction is a direction from distal to proximal; or,
the release tube is the inner tube, and the predetermined direction is a direction from proximal to distal.

In order to achieve the above object, the present invention provides a conveyor, comprising a catheter assembly and the above handle, the catheter assembly comprising an outer tube and an inner tube partially inserted in the outer tube; wherein the handle is connected to a proximal end of the catheter assembly and is configured to control the catheter assembly to cause axial relative movement between the inner tube and the outer tube.

In order to achieve the above object, the present invention also provides a medical device, comprising a medical implant and the above conveyor, wherein the medical implant is loaded at a distal end of the catheter assembly and compressed in a space between the outer tube and the inner tube.

In order to achieve the above object, the present invention also provides a conveyor, comprising a catheter assembly and the above handle, the catheter assembly comprising an outer tube and an inner tube partially inserted in the outer tube, the outer tube or the inner tube used as a release tube; wherein the handle is connected to a proximal end of the catheter assembly and is configured to control the release tube to move in the predetermined direction so as to cause relative movement between the inner tube and the outer tube; the second limiting mechanism is connected to the release tube.

In order to achieve the above object, the present invention also provides a medical device, comprising a medical implant and the above conveyor, wherein the medical implant is loaded at a distal end of the catheter assembly and compressed in a space between the outer tube and the inner tube.

Compared with the prior art, the handle, conveyor and medical device according to the present invention have the following advantages:
The conveyor includes a catheter assembly and a handle. The catheter assembly includes an outer tube and an inner tube partially inserted in the outer tube. Axial relative movement between the inner tube and the outer tube can be caused. The outer tube or the inner tube is used as a release tube. The handle includes a housing, a first driving part and a limiting assembly, wherein the first driving part is for driving the release tube to move in a predetermined direction; the limiting assembly includes a first limiting mechanism and a second limiting mechanism, the first limiting mechanism is partially arranged in the housing and connected to the housing, and the first limiting mechanism is configured to be rotatable around an axis of the release tube; the second limiting mechanism is arranged in the housing and configured to move simultaneously with the release tube; the handle is configured such that, when the first driving part drives the release tube to move in the predetermined direction to a predetermined position, the first limiting mechanism and the second limiting mechanism engage with each other, so that the release tube is prevented from further moving in the predetermined direction; when the first limiting mechanism rotates around the axis of the release tube to cause the first limiting mechanism and the second limiting mechanism to disengage from each other, the release tube is allowed to further move in the predetermined direction. The limiting assembly of the conveyor can be used to temporarily prevent the release tube from further moving in a predetermined direction when the release tube moves to a predetermined position, so as to facilitate the operator to perform other operations and meet usage requirements. Thereafter, the first limiting mechanism can be rotated to cause the first limiting mechanism and the second limiting mechanism to engage with each other, so that the first driving part is allowed to drive the release tube to further move in the predetermined direction. Simple and convenient operations are therefore achieved.

The conveyor includes a catheter assembly, a handle, a first driving part and a limiting assembly. The catheter assembly includes an outer tube and an inner tube partially inserted in the outer tube. The outer tube or the inner tube is used as a release tube. The housing is connected to the proximal end of the catheter assembly. The first driving part is connected to the release tube and is configured to drive the release tube to move in a predetermined direction. The limiting assembly includes a first limiting mechanism and a second limiting mechanism, the first limiting mechanism is partially arranged in the housing and connected to the housing, and the first limiting mechanism is configured to be rotatable around an axis of the housing; the second limiting mechanism is arranged in the housing and connected to the release tube; the conveyor is configured such that, when the release tube moves in the predetermined direction to a predetermined position, the first limiting mechanism and the second limiting mechanism engage with each other, so that the release tube is prevented from further moving in the predetermined direction; when the first limiting mechanism rotates around the axis of the release tube to cause the first limiting mechanism and the second limiting mechanism to disengage from each other, the release tube is allowed to further move in the predetermined direction. The limiting assembly of the conveyor can be used to temporarily prevent the release tube from further moving in a predetermined direction when the release tube moves to a predetermined position, so as to facilitate the operator to perform other operations and meet usage requirements. Thereafter, the first limiting mechanism can be rotated to cause the first limiting mechanism and the second limiting mechanism to engage with each other, so that the first driving part is allowed to drive the release tube to further move in the predetermined direction. Simple and convenient operations are therefore achieved.

### BRIEF DESCRIPTION OF THE DRAWINGS

The present invention will be better understood with reference to the accompanying drawings provided without limiting the invention, in which:
Fig. 1 is a structural schematic diagram of a conveyor according to an embodiment of the present invention;
Fig. 2 is a partial structural schematic diagram of a conveyor according to an embodiment of the present invention;
Fig. 3 is a partial structural schematic diagram of a conveyor according to an embodiment of the present invention, in which part of the housing is omitted to show the structure inside the housing;
Fig. 4 is a partially enlarged view of a conveyor according to an embodiment of the present invention, in which the outer tube is in a state before it moves in a direction from distal to proximal;
Fig. 5 is a partially enlarged view of a conveyor provided according to an embodiment of the present invention, in which the outer tube moves in a direction from distal to proximal to a predetermined position and the first limiting mechanism and the second limiting mechanism engage with each other;
Fig. 6 is a partially enlarged view of a conveyor according to an embodiment of the present invention, in which the first limiting mechanism and the second limiting mechanism are disengaged from each other;
Fig. 7 is a partial enlarged view of a conveyor shown in Fig. 3;
Fig. 8 is a structural schematic diagram of a second connecting component of a conveyor according to an embodiment of the present invention;
Fig. 9 is a structural schematic diagram of a third connecting component of a conveyor according to an embodiment of the present invention;
Fig. 10 is a structural schematic diagram of an anti-rotation member of a conveyor according to an embodiment of the application.

List of reference numerals:
1000, handle; 1101, first fastening ring; 1102, second fastening ring; 1110, housing; 1111, first slide slot; 1113, observation window; 1120, first driving part; 1130, second driving part; 1131, second slide slot; 1210, threaded sleeve; 1220, screw rod; 1310, first limiting mechanism; 1311, first positioning seat; 1312, first limiting part; 1320, second limiting mechanism; 1321, second limiting part; 1313, first lever; 1314, first elastic sleeve; 1410, second positioning seat; 1411, first positioning sub-seat; 1412, connecting arm; 1413, second positioning sub-seat; 1420, connector tube; 1430, third limiting part; 1510, third positioning seat; 1511, first central through hole; 1512, avoidance slot; 1520, second lever; 1530, second elastic sleeve; 1610, auxiliary assembly plate; 1710, anti-rotation member; 1711, second central through hole; 1712, first anti-rotation part; 1713, second anti-rotation part;
2100, outer tube; 2200, inner tube.

### DETAILED DESCRIPTION

The following describes the embodiments of the present invention through specific examples. Those skilled in the art can easily understand other advantages and effects of the present invention from the content disclosed in this specification. The present invention can also be implemented or applied through other different specific embodiments. It should be noted that the diagrams provided in this embodiment only illustrate the basic concept of the present invention in a schematic manner. The drawings only show the components related to the present invention and do not follow the numbers, shapes and dimensions of components in actual implementation, the type, quantity and proportion of each component can be arbitrarily changed, and arrangement of component may also be more complex.

In addition, each embodiment described below has one or more technical features, but this does not mean that the inventor must implement all the technical features in any embodiment at the same time, or can only implement all or some of the technical features in different embodiments separately. In other words, on the premise that implementation is possible, those skilled in the art can, based on the disclosure of the present invention and design specifications or implementation requirements, selectively implement some or all of the technical features in any embodiment, or selectively implement a combination of some or all of the technical features in multiple embodiments, thereby increasing the flexibility of the implementation of the present invention.

As used in this specification, the singular forms "a", "an" and "the" include plural referents, and the plural form "plurality" includes two or more referents unless the content clearly dictates otherwise. As used in this specification, the term "or" is generally used in its sense including "and/or" unless the content clearly dictates otherwise, and the terms "mounted," "connected," and "connected" shall be understood in a broad sense, for example, it can be a fixed connection, a detachable connection, or an integral connection. The connection can be mechanical or electrical. It can be a direct connection or an indirect connection through an intermediary. It can be an internal connection between two elements or an interaction between two elements. For those of ordinary skill in the art, the specific meanings of the above terms in the present invention can be understood according to specific circumstances.

To make the objectives, advantages and features of the present invention clearer, the present invention will be further described in detail below with reference to the figures. It is noted that the figures are provided in a very simplified form not necessarily presented to scale, with their only intention to facilitate convenience and clarity in explaining the disclosed embodiments. Throughout the figures, like reference numerals indicate the same or analogous components or elements.

As used herein, the terms "proximal" and "distal" refer to the relative orientation, relative position, and direction of elements or actions relative to each other from the perspective of an operator using the medical device. Although "proximal" and "distal" are not restrictive, "proximal end" usually refers to the end of the medical device that is close to the operator during normal operation, and "distal end" usually refers to the end that first enters the patient's body, that is, the "proximal end" is the end far away from the patient, and the "distal end" is the end close to the patient. The term "axial" refers to the direction coincident with or parallel to the central axis of the medical device; "radial" refers to the direction perpendicular to the central axis of the medical device.

### EMBODIMENT 1

This embodiment provides a conveyor for conveying and releasing a medical implant to a target position in a patient's body. The medical implant can be but not limited to a medical stent, valve prosthesis or embolization spring coil.

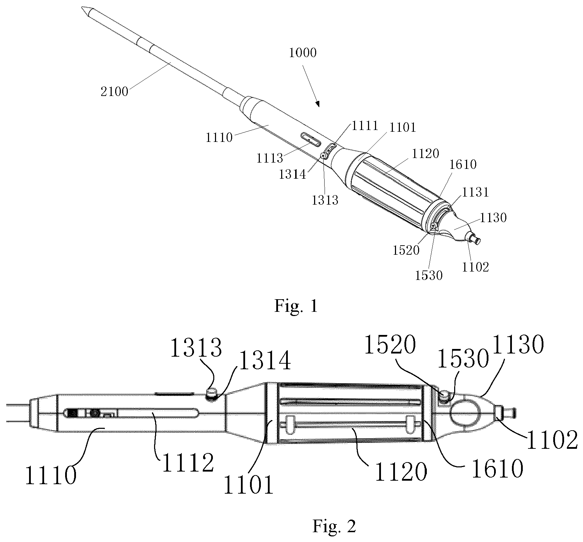

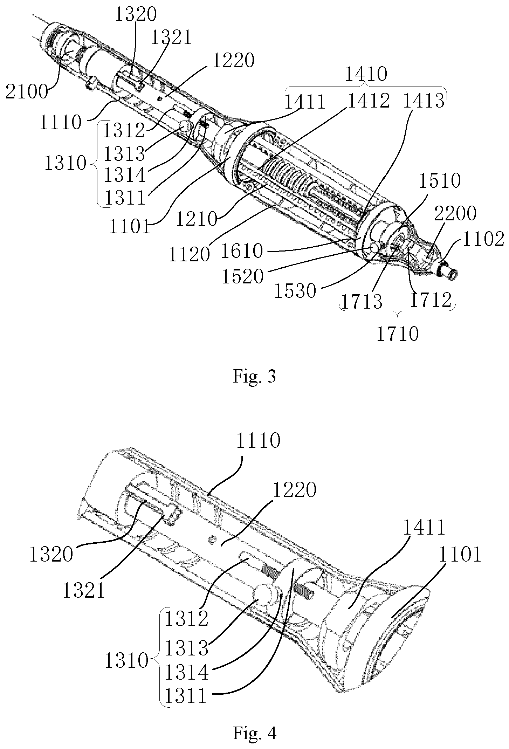

Fig. 1 shows a schematic diagram showing the overall structure of the conveyor, Fig. 2 shows a schematic diagram showing the structural appearance of the handle 1000 of the conveyor, and Fig. 3 shows a structural schematic diagram of the handle 1000 with part of the housing omitted.

Please refer to Figs. 1 to 3, the conveyor includes a handle 1000 and a catheter assembly. The catheter assembly may include an outer tube 2100 and an inner tube 2200 partially inserted in the outer tube 2100, and an axial relative movement can be caused between the outer tube 2100 and the inner tube 2200. The outer tube 2100 or the inner tube 2200 is used as a release tube. When a medical implant is compressed between the outer tube 2100 and the inner tube 2200, the release tube can be operated to move along the axial direction of the handle 1000 to release the medical implant. Specifically, when the outer tube 2100 is used as the release tube, the operator can move back the outer tube 2100 (that is, the outer tube 2100 moves in the direction from distal to proximal) to release the medical implant; when the inner tube 2200 is used as the release tube, the operator can push the inner tube 2200 (that is, the inner tube 2200 moves in the direction from proximal to distal) to release the medical implant. Those skilled in the art will understand that the operator can also operate the release tube to move along the axial direction of the handle 1000 to retrieve the medical implant. In the following description, the outer tube 2100 is used as the release tube as an example, but this should not limit the embodiments of the present invention.

The catheter assembly is used to load a medical implant and convey the medical implant into the patient's body. The handle 1000 is connected to the proximal end of the catheter assembly and is used to control the catheter assembly to cause axial relative movement between the outer tube 2100 and the inner tube 2200 so that the medical implant can be released at a target position in the patient's body. Specifically, the handle 1000 includes a housing 1110, a first driving part 1120 and a limiting assembly. The first driving part 1120 is preferably connected to the housing 1110 and used to drive the outer tube 2100 to move axially relative to the inner tube 2200 to complete the release of the medical implant. The limiting assembly includes a first limiting mechanism 1310 and a second limiting mechanism 1320. The first limiting mechanism 1310 is partially disposed in the housing 1110 and can be connected to the inner wall of the housing 1110, and is configured to be rotatable around the axis of the outer tube 2100. It should be noted that the axis of the housing 1110 may coincide with the axis of the outer tube 2100. The second limiting mechanism 1320 is disposed within the housing and configured to move simultaneously with the outer tube 2100. In a preferred embodiment, the handle further includes the first connecting component that is disposed in the housing 1110, drivingly connected to the first driving part 1120, and used to be connected to the proximal end of the outer tube 2100. In this way, the first connecting component is used to move under the driving of the first driving part 1120 and drive the outer tube 2100 to move axially relative to the inner tube 2200.

The handle 1000 is configured such that when the first driving part 1120 drives the outer tube 2100 to move in a predetermined direction to a predetermined position, the first limiting mechanism 1310 and the second limiting mechanism 1320 engage with each other so that the first driving part 1120 is prevented from driving the outer tube 2100 to further move in the predetermined direction; when the first limiting mechanism 1310 rotates around the axis of the outer tube 2100 to cause the first limiting mechanism 1310 and the second limiting mechanism 1320 to be disengaged from each other, the first driving part 1120 is allowed to drive the outer tube 2100 to further move in the predetermined direction. The predetermined direction is a direction from distal to proximal.

That is to say, when the medical device is conveyed by the conveyor into human body and before the release of the medical device starts, the first limiting mechanism 1310 and the second limiting mechanism 1320 are in a non-engaged state. At this time, the first driving part 1120 can drive the outer tube 2100 to move in a direction from distal to proximal to start the release of medical implant. To simplify the description below, the movement of the outer tube 2100 in the direction from distal to proximal is called the backward movement. When the outer tube 2100 moves to a predetermined position, the first limiting mechanism 1310 and the second limiting mechanism 1320 engage with each other, so that the backward movement of the outer tube 2100 is suspended. At this time, the operator can perform other operations, such as adjusting the posture of the medical implant. After the operator completes the relevant operations, the first limiting mechanism 1310 can be rotated to release the engagement between the first limiting mechanism 1310 and the second limiting mechanism 1320, so that the first driving part 1120 can drive the outer tube 2100 to further move back. In this embodiment, by providing the limiting assembly and selecting the predetermined position according to actual needs during assembly, the operator can suspend the backward movement of the outer tube 2100 at the predetermined position during operation. This ensures the accuracy of the operation and is conducive to the smooth completion of the release of the medical implant.

It should be noted that the first driving part 1120 may be a manual driving mechanism or an electric driving mechanism, which is not limited in the embodiment of the present invention.

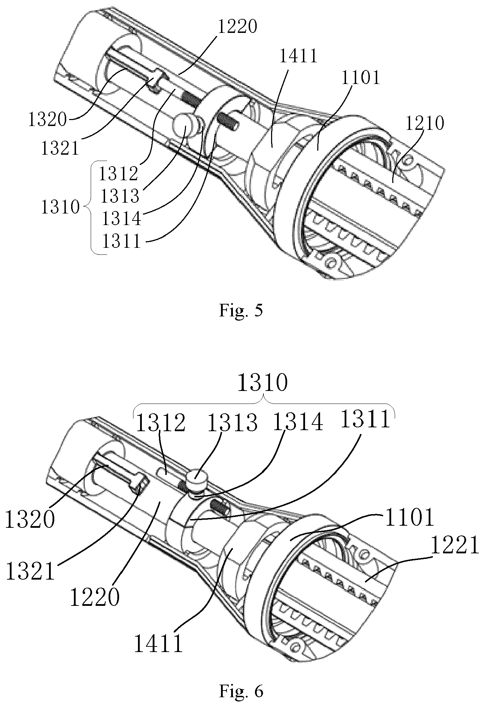

Please focus on Figs. 3 to 6, in an exemplary embodiment, the first limiting mechanism 1310 includes a first positioning seat 1311 and a first limiting part 1312. The first positioning seat 1311 is connected to the inner wall of the housing 1110 and is configured to be rotatable around the axis of the outer tube 2100. The first limiting part 1312 is connected to the first positioning seat 1311 and at least partially protrudes from the distal end face of the first positioning seat 1311, and the first limiting part 1312 moves synchronously with the first positioning seat 1311. The second limiting mechanism 1320 includes a second limiting part 1321. The handle 1000 is configured such that before the release of the medical implant starts, the second limiting part 1321 is located distally to the first limiting part 1312, at least partially aligns with the first limiting part 1312 in the circumferential direction, and is located at a predetermined distance from the first limiting part 1312 (as shown in Fig. 4); when the release of the medical implant starts, the first driving part 1120 first drives the outer tube 2100 to move a predetermined distance back to the predetermined position, and the second limiting part 1321 is in contact with the first limiting part 1312 (as shown in Fig. 5), so that the second limiting mechanism 1320 and the first limiting mechanism 1310 engage with each other; when the first positioning seat 1311 rotates around the axis of the outer tube 2100 and causes the first limiting part 1312 to be staggered with the second limiting part 1321 in the circumferential direction (as shown in Fig. 6), the second limiting mechanism 1320 is disengaged from the first limiting mechanism 1310. It can be understood that if the release tube is implemented as the inner tube, the predetermined direction is the direction from proximal to distal, the first limiting part at least partially protrudes from the proximal end face of the first positioning seat, and the second limiting part is located proximally to the first limiting part (not shown in the figures).

In this embodiment, the first positioning seat 1311 may have an annular structure and have an inner hole. The first connecting component can pass through the inner hole and be connected with the first driving part 1120. To facilitate assembly, the first positioning seat 1311 may include two semicircular positioning blocks connected to each other. Further, the number of the first limiting parts 1312 is multiple, and the multiple first limiting parts 1312 are sequentially arranged along the circumferential direction of the first positioning seat 1311, and the lengths of the portions of the multiple first limiting parts 1312 protruding from the distal end face of the first positioning seat 1311 are sequentially reduced along the first direction, so that along the first direction, the axial distances between the first limiting parts 1312 and the second limiting part are sequentially increased. In this case, the handle 1000 is configured such that when the first positioning seat 1311 rotates in the second direction around the axis of the outer tube 2100 and cause one of the first limiting parts 1312 to be staggered with the second limiting part 1321 in the circumferential direction, another one of the first limiting parts 1312 can at least partially align with the second limiting part 1321 in the circumferential direction. The second direction is opposite to the first direction. The first direction is, for example, a clockwise direction, and the second direction is correspondingly a counterclockwise direction.

In actual operation, the angle that the first positioning seat 1311 rotates each time depends on the number of the first limiting parts 1312 and the distance between two adjacent first limiting parts 1312 in the circumference of the first positioning seat 1311. In one embodiment, the number of the first limiting parts is two (not shown in the figures), which are respectively a first limiting sub-part and a second limiting sub-part. These two limiting sub-parts are spaced apart on the first positioning seat along the first direction, and the angle between the two limiting sub-parts is Φ. When the first driving part drives the outer tube to move back, the second limiting part first contacts the first limiting sub-part. At this time, the operator can rotate the first positioning seat along the second direction around the axis of the outer tube, and the rotation angle of the first positioning seat is Φ, so that the first limiting sub-part is staggered with the second limiting part in the circumferential direction, and the second limiting sub-part at least partially aligns with the second limiting part in the circumferential direction. Thereafter, the first driving part can drive the outer tube to further move back until the second limiting part contacts the second limiting sub-part. Then, the operator can rotate the first positioning seat along the second direction again, so that the second limiting sub-part is staggered with the second limiting part in the circumferential direction. It is understandable that the number of the first limiting parts may be three or more. For the convenience of description, the following description will take the first limiting mechanism 1310 to include only one first limiting part 1312 as an example. However, those skilled in the art can modify the following description to adapt it to the circumstance where the first limiting mechanism 1310 includes two or more first limiting parts 1312.

Preferably, the first limiting part 1312 is movably connected to the first positioning seat 1311 and is configured to be movable along the axial direction of the outer tube 2100. The portion of the first limiting part 1312 protruding from the distal end face of the first positioning seat 1311 can be changed in length to change the predetermined distance between the first limiting part and the second limiting part. The advantage of this arrangement is that when the conveyor is being assembled, the predetermined distance can be adjusted according to for example the specific type and size of the medical implant, thereby enhancing the universal applicability of the conveyor. Optionally, the first positioning seat 1311 has a screw hole extending therethrough along the axial direction thereof, and the first limiting part 1312 may be composed of a screw rod, and the screw rod is inserted into the screw hole. The screw rod is used to form the first limiting part 1312 because the screw rod is simple and convenient to adjust and has a wide adjustment range. Even if the processing error is large, it can be compensated by rotating the screw rod to ensure effective limiting. The first limiting part 1312 can be fixed by set screws to further improve reliability.

Furthermore, the housing 1110 also has thereon a first slide slot 1111 extending along the circumferential direction of the housing. The first limiting mechanism 1310 also includes a first lever 1313. One end of the first lever 1313 is connected to the first positioning seat 1311, and the other end of the first lever 1313 extends out of the housing 1110 through the first slide slot 1111. The first lever 1313 can be used to receive the external force provided by the operator and drive the first positioning seat 1311 to rotate around the axis of the outer tube 2100 to facilitate operation.

Furthermore, the first limiting mechanism 1310 also includes a first elastic sleeve 1314. The first elastic sleeve 1314 is sleeved on the first lever 1313, and is preferably detachably sleeved on the first lever 1313. The outer wall of the first elastic sleeve 1314 contacts the wall of the first slide slot 1111, causing the first elastic sleeve 1314 to deform. That is to say, there is a pressing force between the first elastic sleeve 1314 and the wall of the first slide slot 1111, so that resistance is generated between the first elastic sleeve 1314 and the wall of the first slide slot 1111 when the first lever 1313 is moved under the action of external force to drive the first positioning seat 1311 to rotate. The advantage of this arrangement is that on one hand, it can enhance the operator's feel, and on another hand, the operator needs to provide a certain amount of external force to move the first lever 1313, which avoids misoperation. The resistance of the first lever 1313 when moving can be adjusted by adjusting the size of the first elastic sleeve 1314, and the first elastic sleeve 1314 can be directly replaced after wear, resulting in a convenience in maintenance.

When the first limiting mechanism 1310 includes only one first limiting part 1312, the specific position of the first slide slot 1111 and the length of the first slot 1111 in the circumferential direction of the housing 1110 may depend on the sizes of the first limiting part 1312 and the second limiting part 1321 in the circumferential direction of the housing 1110. Preferably, when the first lever 1313 is located at one end of the first slide slot 1111, the first limiting part 1312 at least partially aligns with the second limiting part 1321 in the circumferential direction, and when the first lever 1313 is located at the other end of the first slide slot 1111, the first limiting part 1312 is staggered with the second limiting part 1321 in the circumferential direction, which facilitates the determination on whether the first positioning seat 1311 is rotated in place, and it is easy to use.

In this embodiment, the first driving part 1120 is, for example, a manual driving mechanism, and is rotatably connected to the proximal end of the housing 1110. When the first driving part 1120 rotates under the action of an external force, the first connecting component moves accordingly and drives the outer tube 2100 to move in the axial direction. To achieve this purpose, please refer back to Fig. 3, the first connecting component includes a threaded sleeve 1210 and a screw rod 1220. The outer wall of the threaded sleeve 1210 is connected to the inner wall of the first driving part 1120 (that is, the threaded sleeve 1210 is located proximally to the first positioning seat 1311), and the threaded sleeve 1210 can be rotated synchronously with the first driving part 1120, the inner wall of the threaded sleeve 1210 is provided with internal threads. The distal end of the screw rod 1220 is connected to the outer tube 2100, and the second limiting mechanism 1320 is connected to the distal outer surface of the screw rod 1220, and the proximal outer surface of the screw rod 1220 is provided with external threads which matches with the internal threads of the threaded sleeve 1210 for spiral transmission. In an alternative embodiment, the inner wall of the first driving part is provided with internal threads, the first connecting component only includes a screw rod, and the external threads on the proximal end of the screw rod matches with the internal threads of the first driving part for spiral transmission (not shown in the figures). It can be understood that the screw rod 1220 can be integrally formed with the second limiting mechanism 1320, or the screw rod 1220 and the second limiting mechanism 1320 can be formed separately and then connected together.

The housing 1110 is also provided with an observation window 1113 extending along the axial direction of the housing. The operator can observe the movement of the screw rod 1220 through the observation window 1113, and thereby check the extent of the movement of the outer tube 2100 and determine the moving direction and distance of the outer tube. Not only that, the screw rod 1220 can also be provided with a scale mark which is visible to the outside through the observation window 1113, so that the operator can know the moving distance of the outer tube 2100 in real time.

Further, please refer back to Figs. 1 to 3, when the outer tube 2100 is used as the release tube, the handle 1000 preferably also includes a second driving part 1130 arranged proximally to the first driving part 1120. The proximal end of the inner tube 2200 extends from the proximal end of the outer tube 2100 to the second driving part 1130, and is directly or indirectly connected to the second driving part 1130, so that the second driving part 1130 can drive the inner tube 2200 to move axially under the action of external force.

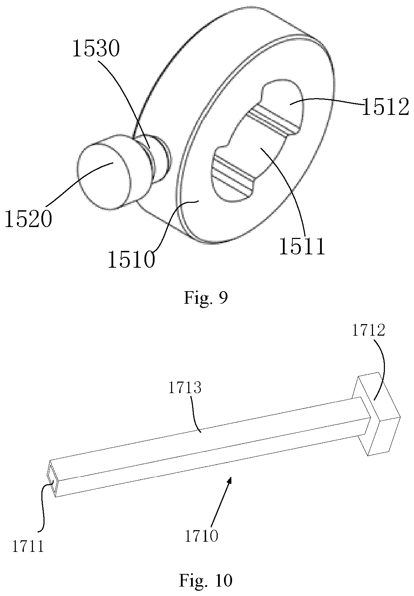

Please focus on Fig. 3 in conjunction with Fig. 9, in a non-limiting embodiment, the handle 1000 further includes a second connecting component and a third connecting component, and the second connecting component is disposed in the housing 1110 and connected to the inner wall of the housing 1110. The third connecting component is partially disposed in the second driving part 1130 and connected to the second driving part 1130. The third connecting component is configured to be rotatable about the axis of the second driving part 1130 and is used to selectively connect with the second connecting component. The handle 1000 is configured such that when the third connecting component is connected to the second connecting component, the second driving part 1130 remains axially stationary relative to the housing 1110 and the first driving part 1120, and the second driving part 1130 is prevented from driving the inner tube 2200 to move axially, when the third connecting component rotates around the axis of the second driving part 1130 and causes the third connecting component and the second connecting component are disconnected from each other, the second driving part 1130 is allowed to move in a direction away from the housing 1110 and the first driving part 1120 under the action of external force and to drive the inner tube 2200 to move in a direction from distal to proximal (that is, to drive the inner tube 2200 to move back). In this way, after the release of the medical implant is completed, the operator can disconnect the third connecting component from the second connecting component, and then move the second driving part 1130 away from the housing 1110, thereby driving the inner tube 2200 to quickly move back into the outer tube 2100. As a result, adverse effects on the patient are reduced. The second driving part 1130 can be driven manually by the operator or driven and moved by an electric device.

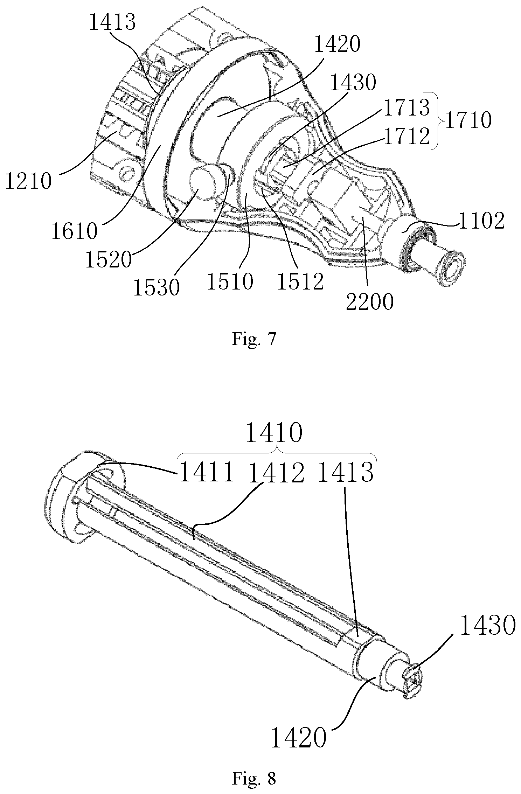

Please focus on Figs. 7 to 9, the second connecting component includes a second positioning seat 1410, a connector tube 1420 and a third limiting part 1430. The second positioning seat 1410 is connected to the inner wall of the housing 1110. The connector tube 1420 is connected to the second positioning seat 1410, and the proximal end of the connector tube 1420 extends toward the second driving part 1130. The third limiting part 1430 is connected to the connector tube 1420, preferably at the proximal end of the connector tube 1420, and extends outward along the radial direction of the connector tube 1420. Here, the third limiting part 1430 extends outward along the radial direction of the connector tube 1420, including the situation where the third limiting part 1430 extends strictly along the radial direction, and the situation where the third limiting part 1430 extends substantially along the radial direction, as long as the third limiting part 1430 protrudes from the outer peripheral surface of the connector tube 1420. The third connecting component includes a third positioning seat 1510 that is movably connected to the inner wall of the second driving part 1130 and is configured to be rotatable around the axis of the second driving part 1130. The third positioning seat 1510 is provided with a first central through hole 1511 and an avoidance slot 1512. The first central through hole 1511 is used for allowing the proximal end of the connector tube 1420 to pass through. The avoidance slot 1512 is connected to the first central through hole 1511 and is used for allowing the third limiting part 1430 to pass through.

When the third limiting part 1430 is located proximally to the third positioning seat 1510 and is staggered with the avoidance slot 1512 in the circumferential direction, and when the third limiting part 1430 is pressed against the third positioning seat 1510, the third connecting component is connected to the second connecting component. At this time, the third limiting part 1430 and the third positioning seat 1510 limit each other so that the second driving part 1130 is prevented from moving in the axial direction under the action of external force, thereby preventing the second driving part 1130 from driving the inner tube 2200 to move axially. When the third positioning seat 1510 rotates around the axis of the second driving part 1130 under the action of external force, until the avoidance slot 1512 at least partially aligns with the third limiting part 1430 in the circumferential direction and allows the third limiting part 1430 to pass through, the third connecting component and the second connecting component are disconnected from each other. At this time, the third limiting part 1430 and the third positioning seat 1510 are released from limiting each other, and no longer hinder the movement of the second driving part 1130 in a direction from distal to proximal. Therefore, the operator can pull back the second driving part 1130 and drive the inner tube 2200 to move back, and at the same time, the third positioning seat 1510 moves in a direction from distal to proximal, and the connector tube 1420 and the third limiting part 1430 pass through the third positioning seat 1510 at the same time to a side distal to the third positioning seat 1510.

It can be understood that when the conveyor is being assembled, the proximal end of the inner tube 2200 passes through the first positioning seat 1311 and the connector tube 1420 to be directly or indirectly connected to the second driving part 1130.

In this embodiment, it is preferred that the shape and size of the avoidance slot 1512 match the shape and size of the third limiting part 1430. Taking Figs. 8 and 9 as an example, the third limiting part 1430 and the avoidance slot 1512 are both fan-shaped, and the avoidance slot 1512 is slightly larger than the third limiting part 1430. Therefore, the third limiting part 1430 can pass through the avoidance slot 1512. In other embodiments, the third limiting part 1430 and the avoidance slot 1512 may be square-shaped, special-shaped or of other shapes. Furthermore, a guide surface (not shown in the figures) is also formed on the distal end of the third limiting part 1430, and the distance from the guide surface to the axis of the connector tube 1420 gradually increases along the direction from distal to proximal. Therefore, the third limiting part 1430 can pass through the avoidance slot 1512 smoothly.

The number of the third limiting parts 1430 is preferably multiple. The multiple third limiting parts 1430 are evenly arranged along the circumference of the connector tube 1420 to improve the force on the third positioning seat 1510 to be evenly distributed when the third connecting component and the second connecting component are connected. Therefore, the service life is improved. The number of the avoidance slots 1512 may be equal to the number of the third limiting parts 1430, and they are arranged correspondingly. As those skilled in the art can understand, the angle that the third positioning seat 1510 rotates to switch the third connecting component and the second connecting component from a connected state to a non-connected state (where they are disconnected from each other) depends on the number and arrangement of the third limiting parts 1430. For example, when the number of the third limiting parts 1430 is two, and the two third limiting parts 1430 are evenly arranged along the circumference of the connector tube 1420, the third positioning seat 1510 can be rotated 90° in a predetermined direction (clockwise or counterclockwise) to switch the third connecting component and the second connecting component from a connected state to a non-connected state.

To facilitate operation, the portion of the second driving part 1130 corresponding to the third positioning seat 1510 defines thereon a second slide slot 1131 extending along the circumferential direction of the second driving part 1130. The third connecting component also includes a second lever 1520. One end of the second lever 1520 is connected to the third positioning seat 1510, and the other end of the second lever 1520 extends out of the second driving part 1130 through the second slide slot 1131 so as to receive the external force exerted by the operator to drive the third positioning seat 1510 to rotate. The position and length of the second slide slot 1131 (that is, the size of the second slide slot 1131 in the circumferential direction of the second driving part 1130) depend on the number and arrangement of the third limiting parts 1430. Generally, when the third connecting component is connected to the second connecting component, the second lever 1520 is located at one end of the second slide slot 1131; when the third connecting component is disconnected from the second connecting component, the second lever 1520 is located at the other end of the second slide slot. This makes it easy to determine whether the third positioning seat 1510 is rotated in place.

Further, the third connecting component further includes a second elastic sleeve 1530. The second elastic sleeve 1530 is preferably detachably sleeved on the second lever 1520, and the outer wall of the second elastic sleeve 1530 is in contact with the wall of the second slide slot 1131, so that the second elastic sleeve 1530 is deformed. In other words, there is a mutual extrusion force between the second elastic sleeve 1530 and the wall of the second slide slot 1131. When the operator applies an external force to move the second lever 1520 along the second slide slot 1131, resistance is generated between the second elastic sleeve 1530 and the wall of the second slide slot 1131. On one hand, this resistance can enhance the operator's feel, and on another hand, the operator needs to provide a certain amount of external force to move the second lever 1520, which avoids misoperation. The resistance of the second lever 1520 when moving can be adjusted by adjusting the size of the second elastic sleeve 1530, and the second elastic sleeve 1530 can be directly replaced after wear, resulting in a convenience in maintenance.

In addition, since the second positioning seat 1410 is connected to the housing 1110, the proximal end of the connector tube 1420 needs to extend to the second driving part 1130, that is to say, the second connecting component extends through the first driving part 1120. In order to prevent the connection between the first connecting component and the first driving part 1120 from being interfered by the second connecting component, the structure of the second connecting component needs to be designed reasonably. Please focus on Fig. 8, the second positioning seat 1410 includes a first positioning sub-seat 1411, a connecting arm 1412 and a second positioning sub-seat 1413. The first positioning sub-seat 1411 is connected to the inner wall of the housing 1110. Two ends of the connecting arm 1412 are connected to the first positioning sub-seat 1411 and the second positioning sub-seat 1413 respectively. The number of the connecting arms 1412 is at least two, preferably two, and the two connecting arms 1412 are arranged at intervals along the circumference of the first positioning sub-seat 1411. The connector tube 1420 is connected to the proximal end face of the second positioning sub-seat 1413. It can be understood that the first positioning sub-seat 1411 and the second positioning sub-seat 1413 are each provided with a channel connected with the inner cavity of the connector tube 1420 for allowing the screw rod 1220 and the inner tube 2200 to pass through (specifically, the proximal end of the screw rod 1220 passes through the channel of the first positioning sub-seat 1411, and the proximal end of the inner tube 2200 passes through the channel of the second positioning sub-seat 1413). When the conveyor is being assembled, the external thread on the screw rod 1220 passes through the gap between the two connecting arms 1412 to match the internal threads on the threaded sleeve 1210 (or on the inner wall of the first driving part 1120).

Preferably, the area of the distal end face of the connector tube 1420 is smaller than the area of the proximal end face of the second positioning sub-seat 1413. Referring to Fig. 7, the handle 1000 further includes an auxiliary assembly plate 1610. The shape of the auxiliary assembly plate 1610 is preferably circular, and a circular second slide slot is defined in the distal end face of the auxiliary assembly plate 1610. The auxiliary assembly plate 1610 is used to be sleeved on the connector tube 1420, and the distal end of the auxiliary assembly plate 1610 is pressed against the proximal end face of the second positioning sub-seat 1413. At the same time, the proximal end of the first driving part 1120 is inserted into the second slide slot and can rotate along the second slide slot under the action of external force. When the third connecting component is connected to the second connecting component, the distal end of the second driving part 1130 is pressed against the proximal end face of the auxiliary assembly plate 1610. Of course, the proximal end face of the auxiliary assembly plate 1610 may be provided with an annular groove that matches the distal end of the second driving part 1130.

Please continue to refer to Fig. 7 in combination with Fig. 10, the handle 1000 further includes a fourth connecting component. The fourth connecting component is connected to the inner wall of the second driving part 1130 and is also used to connect to the proximal end of the inner tube 2200 (that is, the inner tube 2200 is indirectly connected to the second driving part 1130 through the fourth connecting component). Optionally, the fourth connecting component includes an anti-rotation member 1710 having a second central through hole 1711 for allowing the inner tube 2200 to pass through. The anti-rotation member 1710 includes a first anti-rotation part 1712 and a second anti-rotation part 1713. The first anti-rotation part 1712 is connected to the inner wall of the second driving part 1130 and is configured to remain stationary relative to the second driving part 1130 in the circumferential direction, and the second anti-rotation part 1713 is at least partially inserted into the inner cavity of the connector tube 1420 and configured to remain stationary relative to the connector tube 1420 in the circumferential direction. Specifically, the first anti-rotation part 1712 and the second anti-rotation part 1713 each have a non-rotational shape. For example, the cross sections of the first anti-rotation part 1712 and the second anti-rotation part 1713 are rectangle. The shape of the cross section of at least part of the inner cavity of the connector tube 1420 matches the shape of the second anti-rotation part 1713, that is, the cross section of at least part of the inner cavity of the connector tube 1420 is also rectangle. By providing the anti-rotation member 1710, the inner tube 2200 can be prevented from rotating when the operator pulls the second driving part 1130 to drive the inner tube 2200 to move in the direction from distal to proximal.

In addition, the proximal end of the inner tube 2200 passes through the second driving part 1130 and is connected with a Luer connector for connection with a syringe or other external equipment.

It should also be noted that, in order to facilitate the assembly of the conveyor, the housing 1110 has preferably an assembled structure including a first sub-housing and a second sub-housing. The first driving part 1120 and the third driving part 1130 each also have an assembled structure. The first driving part 1120 includes a third sub-housing and a fourth sub-housing. The second driving part 1130 includes a fifth sub-housing and a sixth sub-housing. In addition, the handle 1000 also includes a first fastening ring 1101 and a second fastening ring 1102 (as annotated in Fig. 1). The first fastening ring 1101 is provided between the housing 1110 and the first driving part 1120 to ensure a firm connection between them. The second fastening ring 1102 is provided at the proximal end of the second driving part 1130, so that the fifth sub-housing and the sixth sub-housing are firmly connected.

It should also be noted that the conveyor according to the embodiment of the present invention is provided with an evacuation tube, and reference can be made to the prior art for the arrangement of the evacuation tube.

Furthermore, this embodiment also provides a handle, which is the handle 1000 as mentioned above.

Furthermore, this embodiment also provides a medical device, including a medical implant and the aforementioned conveyor. The medical implant is loaded at the distal end of the catheter assembly and compressed in the space between the inner tube 2200 and the outer tube 2100.

### EMBODIMENT 2

This embodiment provides a conveyor. The difference between this embodiment and Embodiment 1 is that the second limiting mechanism is connected to the release tube.

Furthermore, this embodiment also provides a medical device, including a medical implant and the conveyor. The medical implant is loaded at the distal end of the catheter assembly and compressed in the space between the outer tube and the inner tube.

Although the present invention is disclosed above, it is not limited thereto. Various changes and modifications can be made to the present invention by those skilled in the art without departing from the scope of the invention. Accordingly, the invention is intended to embrace all such modifications and variations if they fall within the scope of the appended claims thereof.

## Claims

1. A handle (1000), for controlling a catheter assembly, the catheter assembly comprising an outer tube (2100) and an inner tube (2200) partially inserted in the outer tube (2100), the outer tube (2100) or the inner tube (2200) used as a release tube; wherein the handle (1000) comprises:
a housing (1110);
a first driving part (1120), for driving the release tube to move in a predetermined direction; and
a limiting assembly, comprising a first limiting mechanism (1310) and a second limiting mechanism (1320), the first limiting mechanism (1310) partially arranged in the housing (1110) and connected to the housing (1110), and the first limiting mechanism (1310) configured to be rotatable around an axis of the release tube; the second limiting mechanism (1320) arranged in the housing (1110) and configured to move with movement of the release tube;
wherein the handle (1000) is configured such that, when the first driving part (1120) drives the release tube to move in the predetermined direction to a predetermined position, the first limiting mechanism (1310) and the second limiting mechanism (1320) engage with each other, so that the release tube is prevented from further moving in the predetermined direction; when the first limiting mechanism (1310) rotates around the axis of the release tube to cause the first limiting mechanism (1310) and the second limiting mechanism (1320) to disengage from each other, the first driving part (1120) is allowed to drive the release tube to further move in the predetermined direction;
wherein the handle (1000) further comprises a first connecting component, wherein the first connecting component is arranged in the housing (1110) and is drivingly connected to the first driving part (1120), and the first connecting component is further configured to connect with a proximal end of the release tube; the second limiting mechanism (1320) is connected to the first connecting component;
and wherein the first limiting mechanism (1310) comprises a first positioning seat (1311) and a first limiting part (1312); wherein the first positioning seat (1311) is connected to the housing (1110) and is configured to be rotatable around the axis of the release tube; the first limiting part (1312) is connected to the first positioning seat (1311) and at least partially protrudes from a predetermined end face of the first positioning seat (1311); the second limiting mechanism (1320) comprises a second limiting part (1321);
wherein the handle (1000) is configured such that when the first limiting part (1312) and the second limiting part (1321) at least partially align with each other in a circumferential direction, and when the first limiting part (1312) and the second limiting part (1321) abut against each other, the first limiting mechanism (1310) and the second limiting mechanism (1320) engage with each other; when the first positioning seat (1311) rotates around the axis of the release tube to cause the first limiting part (1312) and the second limiting part (1321) to be staggered with each other in the circumferential direction, the second limiting mechanism (1320) and the first limiting mechanism (1310) are disengaged from each other.

2. The handle (1000) according to claim 1, wherein the first limiting part (1312) is movably connected to the first positioning seat (1311) and configured to be movable along an axial direction of the release tube to change a length of a portion of the first limiting part (1312) protruding from the predetermined end face of the first positioning seat (1311).

3. The handle (1000) according to claim 2, wherein the first positioning seat (1311) defines therein a screw hole extending through the axial direction of the release tube, and the first limiting part (1312) is a screw rod inserted into the screw hole.

4. The handle (100) according to claim 1, wherein the housing (1110) defines thereon a first slide slot (1111) extending in the circumferential direction; the first limiting mechanism (1310) further comprises a first lever (1313) having one end connected to the first positioning seat (1311) and a further end extending out of the housing (1110) through the first slide slot (1111);
optionally, wherein the first limiting mechanism (1310) further comprises a first elastic sleeve (1314) sleeved on the first lever (1313), and the first elastic sleeve (1314) has an outer wall in contact with a wall of the first slide slot (1111) so that the first elastic sleeve (1314) is deformed.

5. The handle (1000) according to any one of claims 1 to 4, wherein the first positioning seat (1311) has an annular structure defining an inner hole, and a proximal end of the first connecting component passes through the inner hole to connect to the first driving part (1120).

6. The handle (1000) according to claim 5, wherein a number of the first limiting parts (1312) is multiple, and the multiple first limiting parts (1312) are arranged sequentially along a circumference of the first positioning seat (1311), and a length of a portion of each first limiting part (1312) protruding from the predetermined end face of the first positioning seat (1311) is sequentially reduced along a first direction, so that an axial distance from each first limiting part (1312) to the second limiting part (1321) increases sequentially along the first direction;
wherein the handle (1000) is configured such that when the first positioning seat (1311) rotates around the axis of the release tube in a second direction to cause one of the first limiting parts (1312) to be staggered with the second limiting part (1321) in the circumferential direction, another one of the first limiting parts (1312) can at least partially align with the second limiting part (1321) in the circumferential direction; the second direction is opposite to the first direction.

7. The handle (1000) according to any one of claims 1 to 4, wherein the first driving part (1120) is rotatably connected to a proximal end of the housing (1110);
an inner wall of the first driving part (1120) is provided with internal threads, the first connecting component comprises a screw rod (1220), a distal end of the screw rod (1220) is connected to the proximal end of the release tube, a distal outer surface of the screw rod (1220) is connected to the second limiting mechanism (1320), and a proximal outer surface of the screw rod (1220) is provided with external threads matching with the internal threads of the first driving part (1120) for spiral transmission; or,
the first connecting component comprises a threaded sleeve (1210) and a screw rod (1220), the threaded sleeve (1210) is connected to an inner wall of the first driving part (1120) and is configured to rotate synchronously with the first driving part (1120), an inner wall of the threaded sleeve (1210) is provided with internal threads; a distal end of the screw rod (1220) is connected to the proximal end of the release tube, a distal outer surface of the screw rod (1220) is connected to the second limiting mechanism (1320), and a proximal outer surface of the screw rod (1220) is provided with external threads matching with the internal threads of the threaded sleeve (1210) for spiral transmission.

8. The handle (1000) according to claim 7, wherein the housing (1110) is provided with an observation window (1113) for observing movement of the screw rod (1220).

9. The handle (1000) according to any one of claims 1 to 4, wherein the release tube is the outer tube (2100), and the predetermined direction is a direction from distal to proximal; or,
the release tube is the inner tube (2200), and the predetermined direction is a direction from proximal to distal.

10. A conveyor, comprising a catheter assembly and the handle (1000) according to any one of claims 1 to 9, the catheter assembly comprising an outer tube (2100) and an inner tube (2200) partially inserted in the outer tube (2100); wherein the handle (1000) is connected to a proximal end of the catheter assembly and is configured to control the catheter assembly to cause axial relative movement between the inner tube (2200) and the outer tube (2100).

11. A medical device, comprising a medical implant and the conveyor according to claim 10, wherein the medical implant is loaded at a distal end of the catheter assembly and compressed in a space between the outer tube (2100) and the inner tube (2200).

12. A conveyor, comprising a catheter assembly and the handle (1000) according to claim 1, the catheter assembly comprising an outer tube (2100) and an inner tube (2200) partially inserted in the outer tube (2100), the outer tube (2100) or the inner tube (2200) used as a release tube; wherein the handle (1000) is connected to a proximal end of the catheter assembly and is configured to control the release tube to move in the predetermined direction so as to cause relative movement between the inner tube (2200) and the outer tube (2100); the second limiting mechanism (1320) is connected to the release tube.

13. A medical device, comprising a medical implant and the conveyor according to claim 12, wherein the medical implant is loaded at a distal end of the catheter assembly and compressed in a space between the outer tube (2100) and the inner tube (2200).

## Patentansprüche

1. Griff (1000) zum Steuern einer Katheterbaugruppe, wobei die Katheterbaugruppe ein Außenrohr (2100) und ein Innenrohr (2200) umfasst, das teilweise in das Außenrohr (2100) eingesetzt ist, wobei das Außenrohr (2100) oder das Innenrohr (2200) als Freigaberohr verwendet wird; wobei der Griff (1000) umfasst:
ein Gehäuse (1110);
ein erstes Antriebsteil (1120) zum Antreiben des Freigaberohrs so, dass es sich in eine vorbestimmte Richtung bewegt; und
eine Begrenzungsbaugruppe, die einen ersten Begrenzungsmechanismus (1310) und einen zweiten Begrenzungsmechanismus (1320) umfasst, wobei der erste Begrenzungsmechanismus (1310) teilweise in dem Gehäuse (1110) angeordnet und mit dem Gehäuse (1110) verbunden ist und der erste Begrenzungsmechanismus (1310) so konfiguriert ist, dass er um eine Achse des Freigaberohrs drehbar ist; wobei der zweite Begrenzungsmechanismus (1320) in dem Gehäuse (1110) angeordnet und so konfiguriert ist, dass er sich mit der Bewegung des Freigaberohrs bewegt;
wobei der Griff (1000) derart konfiguriert ist, dass, wenn das erste Antriebsteil (1120) das Freigaberohr so antreibt, dass es sich in der vorbestimmten Richtung zu einer vorbestimmten Position bewegt, der erste Begrenzungsmechanismus (1310) und der zweite Begrenzungsmechanismus (1320) ineinander einrücken, sodass das Freigaberohr daran gehindert wird, sich weiter in der vorbestimmten Richtung zu bewegen; wenn sich der erste Begrenzungsmechanismus (1310) um die Achse des Freigaberohrs dreht, um zu bewirken, dass der erste Begrenzungsmechanismus (1310) und der zweite Begrenzungsmechanismus (1320) auseinander ausrücken, es dem ersten Antriebsteil (1120) ermöglicht wird, das Freigaberohr so anzutreiben, dass es sich weiter in der vorbestimmten Richtung bewegt;
wobei der Griff (1000) weiter eine erste Verbindungskomponente umfasst, wobei die erste Verbindungskomponente in dem Gehäuse (1110) angeordnet und antriebsmäßig mit dem ersten Antriebsteil (1120) verbunden ist und die erste Verbindungskomponente weiter so konfiguriert ist, dass sie eine Verbindung mit einem proximalen Ende des Freigaberohrs herstellt; wobei der zweite Begrenzungsmechanismus (1320) mit der ersten Verbindungskomponente verbunden ist;
und wobei der erste Begrenzungsmechanismus (1310) einen ersten Positioniersitz (1311) und ein erstes Begrenzungsteil (1312) umfasst; wobei der erste Positioniersitz (1311) mit dem Gehäuse (1110) verbunden und so konfiguriert ist, dass er um die Achse des Freigaberohrs drehbar ist; das erste Begrenzungsteil (1312) mit dem ersten Positioniersitz (1311) verbunden ist und mindestens teilweise von einer vorbestimmten Stirnseite des ersten Positioniersitzes (1311) vorspringt; der zweite Begrenzungsmechanismus (1320) ein zweites Begrenzungsteil (1321) umfasst;
wobei der Griff (1000) derart konfiguriert ist, dass, wenn das erste Begrenzungsteil (1312) und das zweite Begrenzungsteil (1321) in einer Umfangsrichtung mindestens teilweise miteinander fluchten und wenn das erste Begrenzungsteil (1312) und das zweite Begrenzungsteil (1321) aneinander anliegen, der erste Begrenzungsmechanismus (1310) und der zweite Begrenzungsmechanismus (1320) ineinander einrücken; wenn sich der erste Positioniersitz (1311) um die Achse des Freigaberohrs dreht, um zu bewirken, dass das erste Begrenzungsteil (1312) und das zweite Begrenzungsteil (1321) in der Umfangsrichtung zueinander versetzt werden, der zweite Begrenzungsmechanismus (1320) und der erste Begrenzungsmechanismus (1310) auseinander ausrücken.

2. Griff (1000) nach Anspruch 1, wobei das erste Begrenzungsteil (1312) beweglich mit dem ersten Positioniersitz (1311) verbunden und so konfiguriert ist, dass es entlang einer Axialrichtung des Freigaberohrs bewegbar ist, um eine Länge eines Abschnitts des ersten Begrenzungsteils (1312), der von der vorbestimmten Stirnseite des ersten Positioniersitzes (1311) vorspringt, zu verändern.

3. Griff (1000) nach Anspruch 2, wobei der erste Positioniersitz (1311) in sich ein Schraubenloch definiert, das sich durch die Axialrichtung des Freigaberohrs erstreckt, und das erste Begrenzungsteil (1312) eine in das Schraubenloch eingesetzte Schraubenstange ist.

4. Griff (100) nach Anspruch 1, wobei das Gehäuse (1110) an sich einen ersten Gleitschlitz (1111) definiert, der sich in der Umfangsrichtung erstreckt; der erste Begrenzungsmechanismus (1310) weiter einen ersten Hebel (1313) umfasst, von dem ein Ende mit dem ersten Positioniersitz (1311) verbunden ist und ein weiteres Ende sich durch den ersten Gleitschlitz (1111) aus dem Gehäuse (1110) heraus erstreckt;
wahlweise, wobei der erste Begrenzungsmechanismus (1310) weiter eine erste elastische Hülse (1314) umfasst, die auf den ersten Hebel (1313) aufgeschoben ist, und die erste elastische Hülse (1314) eine Außenwand aufweist, die mit einer Wand des ersten Gleitschlitzes (1111) in Kontakt steht, sodass die erste elastische Hülse (1314) verformt wird.

5. Griff (1000) nach einem der Ansprüche 1 bis 4, wobei der erste Positioniersitz (1311) eine ringförmige Struktur aufweist, die ein Innenloch definiert, und ein proximales Ende der ersten Verbindungskomponente durch das Innenloch verläuft, um eine Verbindung zu dem ersten Antriebsteil (1120) herzustellen.

6. Griff (1000) nach Anspruch 5, wobei eine Anzahl der ersten Begrenzungsteile (1312) mehrere beträgt und die mehreren ersten Begrenzungsteile (1312) nacheinander entlang eines Umfangs des ersten Positioniersitzes (1311) angeordnet sind, und eine Länge eines Abschnitts jedes ersten Begrenzungsteils (1312), der von der vorbestimmten Stirnseite des ersten Positionierungssitzes (1311) vorspringt, entlang einer ersten Richtung nach und nach abnimmt, sodass ein axialer Abstand von jedem ersten Begrenzungsteil (1312) zu dem zweiten Begrenzungsteil (1321) entlang der ersten Richtung nach und nach zunimmt;
wobei der Griff (1000) derart konfiguriert ist, dass, wenn sich der erste Positioniersitz (1311) um die Achse des Freigaberohrs in einer zweiten Richtung dreht, um zu bewirken, dass eines der ersten Begrenzungsteile (1312) gegenüber dem zweiten Begrenzungsteil (1321) in Umfangsrichtung versetzt wird, sich ein anderes der ersten Begrenzungsteile (1312) in Umfangsrichtung mindestens teilweise mit dem zweiten Begrenzungsteil (1321) in Flucht bringen kann; wobei die zweite Richtung der ersten Richtung entgegengesetzt ist.

7. Griff (1000) nach einem der Ansprüche 1 bis 4, wobei das erste Antriebsteil (1120) drehbar mit einem proximalen Ende des Gehäuses (1110) verbunden ist;
eine Innenwand des ersten Antriebsteils (1120) mit einem Innengewinde versehen ist, die erste Verbindungskomponente eine Schraubenstange (1220) umfasst, ein distales Ende der Schraubenstange (1220) mit dem proximalen Ende des Freigaberohrs verbunden ist, eine distale Außenfläche der Schraubenstange (1220) mit dem zweiten Begrenzungsmechanismus (1320) verbunden ist, und eine proximale Außenfläche der Schraubenstange (1220) mit einem Außengewinde versehen ist, das zur spiralförmigen Kraftübertragung mit dem Innengewinde des ersten Antriebsteils (1120) zusammenpasst; oder
die erste Verbindungskomponente eine Gewindehülse (1210) und eine Schraubenstange (1220) umfasst, die Gewindehülse (1210) mit einer Innenwand des ersten Antriebsteils (1120) verbunden und so konfiguriert ist, dass sie sich synchron mit dem ersten Antriebsteil (1120) dreht, eine Innenwand der Gewindehülse (1210) mit einem Innengewinde versehen ist; ein distales Ende der Schraubenstange (1220) mit dem proximalen Ende des Freigaberohrs verbunden ist, eine distale Außenfläche der Schraubenstange (1220) mit dem zweiten Begrenzungsmechanismus (1320) verbunden ist, und eine proximale Außenfläche der Schraubenstange (1220) mit einem Außengewinde versehen ist, das zur spiralförmigen Kraftübertragung mit dem Innengewinde der Gewindehülse (1210) zusammenpasst.

8. Griff (1000) nach Anspruch 7, wobei das Gehäuse (1110) mit einem Beobachtungsfenster (1113) zum Beobachten der Bewegung der Schraubenstange (1220) versehen ist.

9. Griff (1000) nach einem der Ansprüche 1 bis 4, wobei das Freigaberohr das Außenrohr (2100) ist und die vorbestimmte Richtung eine Richtung von distal nach proximal ist; oder
das Freigaberohr das Innenrohr (2200) ist und die vorbestimmte Richtung eine Richtung von proximal nach distal ist.

10. Förderer, der eine Katheterbaugruppe und den Griff (1000) nach einem der Ansprüche 1 bis 9 umfasst, wobei die Katheterbaugruppe ein Außenrohr (2100) und ein Innenrohr (2200) umfasst, das teilweise in das Außenrohr (2100) eingesetzt ist; wobei der Griff (1000) mit einem proximalen Ende der Katheterbaugruppe verbunden und so konfiguriert ist, dass er die Katheterbaugruppe steuert, um axiale Relativbewegung zwischen dem Innenrohr (2200) und dem Außenrohr (2100) zu bewirken.

11. Medizinische Vorrichtung, die ein medizinisches Implantat und den Förderer nach Anspruch 10 umfasst, wobei das medizinische Implantat an einem distalen Ende der Katheterbaugruppe geladen und in einem Raum zwischen dem Außenrohr (2100) und dem Innenrohr (2200) komprimiert ist.

12. Förderer, der eine Katheterbaugruppe und den Griff (1000) nach Anspruch 1 umfasst, wobei die Katheterbaugruppe ein Außenrohr (2100) und ein Innenrohr (2200) umfasst, das teilweise in das Außenrohr (2100) eingesetzt ist, das Außenrohr (2100) oder das Innenrohr (2200) als Freigaberohr verwendet wird; wobei der Griff (1000) mit einem proximalen Ende der Katheterbaugruppe verbunden und so konfiguriert ist, dass er das Freigaberohr so steuert, dass es sich in der vorbestimmten Richtung bewegt, um eine Relativbewegung zwischen dem Innenrohr (2200) und dem Außenrohr (2100) zu bewirken; der zweite Begrenzungsmechanismus (1320) mit dem Freigaberohr verbunden ist.

13. Medizinische Vorrichtung, die ein medizinisches Implantat und den Förderer nach Anspruch 12 umfasst, wobei das medizinische Implantat an einem distalen Ende der Katheterbaugruppe geladen und in einem Raum zwischen dem Außenrohr (2100) und dem Innenrohr (2200) komprimiert ist.

## Revendications

1. Poignée (1000), destinée à commander un ensemble cathéter, l'ensemble cathéter comprenant un tube extérieur (2100) et un tube intérieur (2200) inséré partiellement dans le tube extérieur (2100), le tube extérieur (2100) ou le tube intérieur (2200) étant utilisé comme un tube de libération ; dans laquelle la poignée (1000) comprend :
un logement (1110) ;
une première partie d'entraînement (1120), destinée à entraîner le tube de libération à se déplacer dans une direction prédéterminée ; et
un ensemble de limitation, comprenant un premier mécanisme de limitation (1310) et un second mécanisme de limitation (1320), le premier mécanisme de limitation (1310) étant agencé partiellement dans le logement (1110) et relié au logement (1110), et le premier mécanisme de limitation (1310) étant configuré pour pouvoir tourner autour d'un axe du tube de libération ; le second mécanisme de limitation (1320) étant agencé dans le logement (1110) et configuré pour se déplacer avec le mouvement du tube de libération ;
dans laquelle la poignée (1000) est configurée de sorte que, lorsque la première partie d'entraînement (1120) entraîne le tube de libération à se déplacer dans la direction prédéterminée jusqu'à une position prédéterminée, le premier mécanisme de limitation (1310) et le second mécanisme de limitation (1320) entrent en prise l'un avec l'autre, empêchant ainsi le tube de libération de se déplacer davantage dans la direction prédéterminée ; lorsque le premier mécanisme de limitation (1310) tourne autour de l'axe du tube de libération pour amener le premier mécanisme de limitation (1310) et le second mécanisme de limitation (1320) à se désolidariser l'un de l'autre, la première partie d'entraînement (1120) est autorisée à entraîner le tube de libération pour qu'il se déplace davantage dans la direction prédéterminée ;
dans laquelle la poignée (1000) comprend en outre un premier élément de liaison, dans lequel le premier composant de liaison est agencé dans le logement (1110) et est relié par entraînement à la première partie d'entraînement (1120), et le premier composant de liaison est configuré en outre pour être relié à une extrémité proximale du tube de libération ; le second mécanisme de limitation (1320) est relié au premier composant de liaison ;
et dans laquelle le premier mécanisme de limitation (1310) comprend un premier siège de positionnement (1311) et une première partie de limitation (1312) ; dans lequel le premier siège de positionnement (1311) est relié au logement (1110) et est configuré pour tourner autour de l'axe du tube de libération ; la première partie de limitation (1312) est reliée au premier siège de positionnement (1311) et fait au moins partiellement saillie depuis une face d'extrémité prédéterminée du premier siège de positionnement (1311) ; le second mécanisme de limitation (1320) comprend une seconde partie de limitation (1321) ;
dans laquelle la poignée (1000) est configurée de sorte que lorsque la première partie de limitation (1312) et la seconde partie de limitation (1321) s'alignent au moins partiellement l'une avec l'autre dans une direction circonférentielle, et lorsque la première partie de limitation (1312) et la seconde partie de limitation (1321) butent l'une contre l'autre, le premier mécanisme de limitation (1310) et le second mécanisme de limitation (1320) entrent en prise l'un avec l'autre ; lorsque le premier siège de positionnement (1311) tourne autour de l'axe du tube de libération pour amener la première partie de limitation (1312) et la seconde partie de limitation (1321) à être décalées l'une par rapport à l'autre dans la direction circonférentielle, le second mécanisme de limitation (1320) et le premier mécanisme de limitation (1310) sont désolidarisés l'un de l'autre.

2. Poignée (1000) selon la revendication 1, dans laquelle la première partie de limitation (1312) est reliée de manière mobile au premier siège de positionnement (1311) et configurée pour être mobile le long d'une direction axiale du tube de libération afin de modifier la longueur d'une portion de la première partie de limitation (1312) qui fait saillie depuis la face d'extrémité prédéterminée du premier siège de positionnement (1311).

3. Poignée (1000) selon la revendication 2, dans laquelle le premier siège de positionnement (1311) définit dans celui-ci un trou de vis s'étendant à travers la direction axiale du tube de libération, et la première partie de limitation (1312) est une tige filetée insérée dans le trou de vis.

4. Poignée (100) selon la revendication 1, dans laquelle le logement (1110) définit sur celui-ci une première fente de coulissement (1111) s'étendant dans la direction circonférentielle ; le premier mécanisme de limitation (1310) comprend en outre un premier levier (1313) dont une extrémité est reliée au premier siège de positionnement (1311) et une autre extrémité s'étendant hors du logement (1110) à travers la première fente de coulissement (1111) ;
facultativement, dans laquelle le premier mécanisme de limitation (1310) comprend en outre un premier manchon élastique (1314) emmanché sur le premier levier (1313), et le premier manchon élastique (1314) présente une paroi extérieure en contact avec une paroi de la première fente de coulissement (1111) afin que le premier manchon élastique (1314) soit déformé.

5. Poignée (1000) selon l'une quelconque des revendications 1 à 4, dans laquelle le premier siège de positionnement (1311) présente une structure annulaire définissant un trou intérieur, et une extrémité proximale du premier composant de liaison passe à travers le trou intérieur de manière à être reliée à la première partie d'entraînement (1120).

6. Poignée (1000) selon la revendication 5, dans laquelle un certain nombre des premières parties de limitation (1312) est multiple, et les multiples premières parties de limitation (1312) sont agencées séquentiellement le long d'une circonférence du premier siège de positionnement (1311), et la longueur d'une portion de chaque première partie de limitation (1312) faisant saillie depuis la face d'extrémité prédéterminée du premier siège de positionnement (1311) est réduite séquentiellement le long d'une première direction, afin qu'une distance axiale allant de chaque première partie de limitation (1312) à la seconde partie de limitation (1321) augmente séquentiellement le long de la première direction ;
dans laquelle la poignée (1000) est configurée de sorte que lorsque le premier siège de positionnement (1311) tourne autour de l'axe du tube de libération dans une seconde direction pour amener l'une des premières parties de limitation (1312) à être décalée par rapport à la seconde partie de limitation (1321) dans la direction circonférentielle, une autre des premières parties de limitation (1312) peut au moins s'aligner partiellement avec la seconde partie de limitation (1321) dans la direction circonférentielle ; la seconde direction est opposée à la première direction.

7. Poignée (1000) selon l'une quelconque des revendications 1 à 4, dans laquelle la première partie d'entraînement (1120) est reliée de manière à pouvoir tourner à une extrémité proximale du logement (1110) ;
une paroi intérieure de la première partie d'entraînement (1120) est munie de filetages intérieurs, le premier composant de liaison comprend une tige filetée (1220), une extrémité distale de la tige filetée (1220) est reliée à l'extrémité proximale du tube de libération, une surface extérieure distale de la tige filetée (1220) est reliée au second mécanisme de limitation (1320), et une surface extérieure proximale de la tige filetée (1220) est munie de filetages extérieurs correspondant aux filetages intérieurs de la première partie d'entraînement (1120) pour une transmission en spirale ; ou,
le premier composant de liaison comprend un manchon fileté (1210) et une tige filetée (1220), le manchon fileté (1210) est relié à une paroi intérieure de la première partie d'entraînement (1120) et est configuré pour tourner de manière synchrone avec la première partie d'entraînement (1120), une paroi intérieure du manchon fileté (1210) est munie de filetages intérieurs ; l'extrémité distale de la tige filetée (1220) est reliée à l'extrémité proximale du tube de libération, la surface extérieure distale de la tige filetée (1220) est reliée au second mécanisme de limitation (1320), et une surface extérieure proximale de la tige filetée (1220) est munie de filetages extérieurs correspondant aux filetages intérieurs du manchon fileté (1210) pour une transmission en spirale.

8. Poignée (1000) selon la revendication 7, dans laquelle le logement (1110) est muni d'une fenêtre d'observation (1113) pour observer le mouvement de la tige filetée (1220).

9. Poignée (1000) selon l'une quelconque des revendications 1 à 4, dans laquelle le tube de libération est le tube extérieur (2100), et la direction prédéterminée est une direction allant de distal à proximal ; ou,
le tube de libération est le tube intérieur (2200), et la direction prédéterminée est une direction allant de proximal à distal.

10. Transporteur, comprenant un ensemble cathéter et la poignée (1000) selon l'une quelconque des revendications 1 à 9, l'ensemble cathéter comprenant un tube extérieur (2100) et un tube intérieur (2200) inséré partiellement dans le tube extérieur (2100) ; dans lequel la poignée (1000) est reliée à une extrémité proximale de l'ensemble cathéter et est configurée pour commander l'ensemble cathéter pour amener un mouvement relatif axial entre le tube intérieur (2200) et le tube extérieur (2100).

11. Dispositif médical, comprenant un implant médical et le transporteur selon la revendication 10, dans lequel l'implant médical est chargé à une extrémité distale de l'ensemble cathéter et comprimé dans un espace entre le tube extérieur (2100) et le tube intérieur (2200).

12. Transporteur, comprenant un ensemble cathéter et la poignée (1000) selon la revendication 1, l'ensemble cathéter comprenant un tube extérieur (2100) et un tube intérieur (2200) inséré partiellement dans le tube extérieur (2100), le tube extérieur (2100) ou le tube intérieur (2200) étant utilisé comme tube de libération ; dans lequel la poignée (1000) est reliée à une extrémité proximale de l'ensemble cathéter et est configurée pour commander le déplacement du tube de libération dans la direction prédéterminée pour amener un mouvement relatif entre le tube intérieur (2200) et le tube extérieur (2100) ; le second mécanisme de limitation (1320) est relié au tube de libération.

13. Dispositif médical, comprenant un implant médical et le transporteur selon la revendication 12, dans lequel l'implant médical est chargé à une extrémité distale de l'ensemble cathéter et comprimé dans un espace entre le tube extérieur (2100) et le tube intérieur (2200).
